Europäisches Patentamt

European Patent Office

Office européen des brevets

(11) Publication number: 0 319 639
A1

## EUROPEAN PATENT APPLICATION

(21) Application number: 88106221.0

(22) Date of filing: 19.04.88

(51) Int. Cl.⁴: A61C 9/00

(30) Priority: 07.12.87 US 129070

(43) Date of publication of application:
14.06.89 Bulletin 89/24

(84) Designated Contracting States:
AT BE CH DE ES FR GB GR IT LI LU NL SE

(71) Applicant: Colin, Lawrence, Dr.
Box 301
Cross River New York(US)

Applicant: Spehar, Edward R.
40 Orion Way
Neshanic Station New Jersey(US)

(72) Inventor: Colin, Lawrence, Dr.
Box 301
Cross River New York(US)
Inventor: Spehar, Edward R.
40 Orion Way
Neshanic Station New Jersey(US)

(74) Representative: Kinzebach, Werner, Dr. et al
Patentanwälte Reitstötter, Kinzebach und
Partner Sternwartstrasse 4 Postfach 86 06 49
D-8000 München 86(DE)

(54) Dispensing mixer for the storage and mixing of separate materials.

(57) A portable dispensing mixer (10) for intermixing and dispensing, in sequence, a unit dosage of elastomeric impression materials from a common nozzle (38) so as to permit a dental impression to be taken under aseptic conditions. The dispenser includes a syringe (12) which is removably locked to a nozzle assembly (14) containing a mixing element (37).

FIG. 1

# DISPENSING MIXER FOR THE STORAGE AND MIXING OF SEPARATE MATERIALS

## FIELD OF INVENTION

This invention relates to a portable device for intermixing and dispensing two contiguous streams of material independent of their relative viscosity, so as to permit a dental impression to be taken under aseptic conditions in the preparation of a dental restoration.

## BACKGROUND OF THE INVENTION

Commercial devices are readily available for intermixing separate materials and dispensing a dental mixed product through a common spout. The devices vary based upon the composition of the materials to be mixed, their relative viscosity and the application for the intermixed product.

Dental impressions of prepared and unprepared teeth are a vital step in the fabrication of the prosthetic replacement. Such devices as inlays, onlays, full crowns and bridges as well as removable prosthetic devices require replication with accuracy. By means of an impression or negative duplication of the intraoral site, a dental laboratory can convert this reverse likeness to a positive of the original condition. The materials used for taking the impression have improved and presently the use of elastomers is considered the most accurate, stable and easily handled of the impression materials.

A dental impression tray is usually selected for the particular area of the mouth where the impression is to be taken. To obtain an impression of the teeth and surrounding soft tissue, it is desirable to coat the intraoral site with a low viscosity impression material followed by a separate application of a higher viscosity material. The low viscosity material fills the internal critical surfaces of the oral cavity to provide maximum detail, whereas the higher viscosity material serves as a bulking agent between the low viscosity material and the tray. Additionally, the higher viscosity material serves to develop hydraulic pressure to force the lower viscosity impression material into tight apposition to the oral tissue and hard tooth structure.

Presently, the elastomeric impression materials are blended and premixed by hand from separate containers of catalyst and base with the mixed higher viscosity material added to the tray and separately formed lower viscosity material drawn into a syringe. Not only is the blending of each of the impression materials subject to inconsistency from one operation to another, but the overall operation is both messy and time-consuming, and is, in general, performed in the full view of the patient without regard to asepsis.

The dispenser of the present invention can dispense a plurality of different viscosity elastomeric impression materials from a single source in a predetermined sequence and in a predetermined unit dosage for use in taking a dental impression. In using the apparatus of the present invention there is no mess and the procedure is inherently aseptic.

The device of the present invention includes a syringe with multiple compartments for storing separate polymeric materials, a common discharge nozzle for all of the compartments, and a conventional static mixing device made from a multiplicity of twisted auger-like mixing elements or blades. The static mixing element is arbitrarily dropped into the common discharge nozzle without establishing any preset or prefixed alignment. Upon feeding material from the syringe into the common discharge nozzle, the static mixing element is caused to rotate in response to the ingress of impression material into a fixed position against a stop. The static mixing element remains immobile in this fixed position for as long as material is being fed into the discharge nozzle. The dispensing mixer of the present invention may also include a separate diaphragm member for each compartment to prevent leakage between compartments during storage. The diaphragm members are designed to open in response to a predetermined amount of pressure under the control of the operator. Accordingly, the device is simple in design and does not require the static mixing element to be held in a preset rotationally aligned position to assure satisfactory mixing. The volumetric ratio between the two streams of material may be adjusted by varying the size of the syringe compartments.

Another feature of the dispensing mixer of the present invention relates to the removable coupling between the syringe and the common discharge nozzle which permits the nozzle and syringe to be separated for replacement of a fresh charge of material.

Accordingly, it is the principle object of the present invention to provide a mixing dispenser for intermixing a plurality of different viscosity materials from a single source and in a predetermined unit dosage for use in taking a dental impression.

It is another object to provide a mixing dispenser for intermixing a plurality of different viscosity materials through a single common spout which locks in one position and is removably connected to the dispenser.


## BRIEF DESCRIPTION OF DRAWINGS


The configurations which best illustrate the invention is shown in the accompanying drawings, which are to be considered as exemplary rather than limiting, and wherein:

Figure 1 is an exploded view in perspective of the material storage and dispensing mixer of the present invention;

Figure 2 is a cross-sectional view of one of the syringe compartments taken along the lines 2-2 of Figure 1;

Figure 3 is a cross-sectional view similar to Figure 2 showing the syringe of Figure 2 in a partially discharged position;

Figure 4 is a view from the rear of the nozzle assembly of the mixer of Figure 1 taken along the lines 4-4 showing the static mixing device in its assembled position in the nozzle assembly prior to use of the mixer;

Figure 5 is another view similar to Figure 4 showing the static mixing device rotated into a locked position in response to the ingress of storage material fed from the syringe;

Figure 6 shows an alternate embodiment of the syringe of Figure 1;

Figure 7 is a view in perspective of a variation of the embodiment of Figure 6;

Figure 8 is an exploded view of the dispenser of Figure 1 employing a preferred arrangement for removably coupling the syringe and nozzle assembly;

Figure 8A is a cross-sectional view of the dispenser of Figure 8 taken along the lines 8A-8A of Figure 8;

Figure 8B is a cross-sectional view of the dispenser of Figure 8 taken along the lines 8B-8B of Figure 8;

Figure 9 is a side elevation in cross section of the dispensing apparatus of Figure 8 for dispensing multiple elastomeric materials in sequence from a single unit;

Figure 10 is a plan view of a separating diaphragm for the dispenser of Figure 9; and

Figure 11 is a plan view of another separating diaphragm for separating the impression materials of Figure 9.


## DETAILED DESCRIPTION


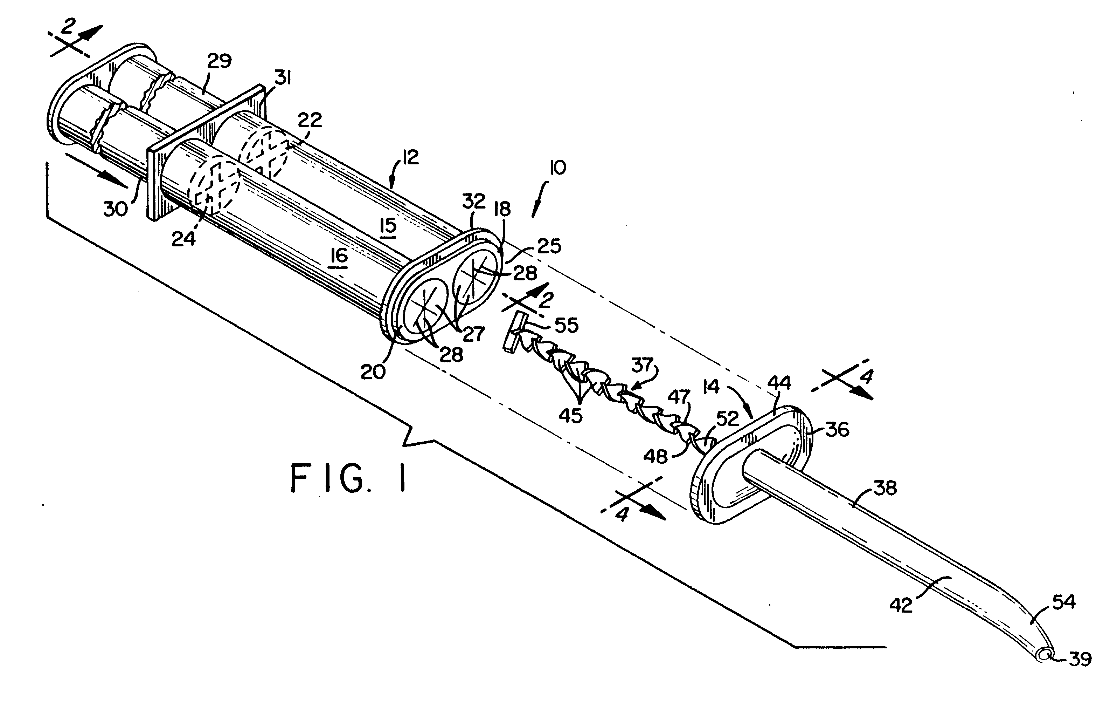

Referring now to Figure 1 showing an exploded view in perspective of the dispensing mixer of the present invention, the dispensing mixer is identified by the reference numeral 10 and comprises in combination, a syringe (12) and a nozzle assembly (14). The syringe (12) is formed from any moldable plastic composition, preferably polystyrene, having two compartments (15) and (16) for separately storing preselected materials to be combined, intermixed and discharged from the nozzle assembly (14). The compartments (15) and (16) may be of any configuration, preferably cylindrical and in the embodiment of Figure 1, are of equal size with each forming a hollow chamber having a predetermined fixed volume. The storage compartments (15) and (16) are adapted to be filled with any material composition of any desired viscosity based on the application for the mixed product.

Each compartment (15) and (16) preferably includes a diaphragm member (18) and (20) and a sealing cap (22) and (24), respectively. The diaphragm members (18) and (20) are in the form of thin cylindrical disks fixedly mounted within the compartments (15) and (16) at the proximal end (25) of the syringe (12). Each diaphragm member (18) and (20) has a plurality of score lines (27) which penetrate through a substantial portion of the thickness of the diaphragm members for forming a multiplicity of pie-shaped sections (28). Upon the application of sufficient pressure, the sections (28) open up to permit the discharge of material from the compartments (15) and (16) past the diaphragm members (18) and (20) into the nozzle assembly (14).

The stored materials are driven simultaneously from the compartments (15) and (16). Any conventional drive mechanism such as a pair of pistons (29) and (30) may be used for this purpose. The pistons (29) and (30) are coupled together to be driven in unison using, e.g., a conventional double-barreled ratchet-type caulking gun (not shown), which may be either mechanically and automatically actuated.

The diaphragm members (18) and (20) may be fixedly mounted in each compartment (15) and (16) at the proximal end (25) of the syringe by press fitting each diaphragm member into a compartment and ultrasonically welding it to the compartment or simply by applying an adhesive between the diaphragm members (18) and (20) and the compartment walls. The diaphragm members (18) and (20) should be very thin in order to permit the sections (28) to exhibit a natural resiliency or tendency to self-lock and return to its normally closed position after the applied force from the pistons (29) and (30) has been removed. The preferred material for the diaphragm members (18) and (20) is polyethylene, although a thin metallic material may equally be used. The self-locking nature of the diaphragm members (18) and (20) permits the syringe (12) to be used repeatedly or intermittently without emptying the stored contents of each compartment (15) and (16) during each use. The self-locking diaphragm members (18) and (20) effectively reclose each compartment (15) and (161 to prevent any remaining material from intermixing, curing and solidifying.

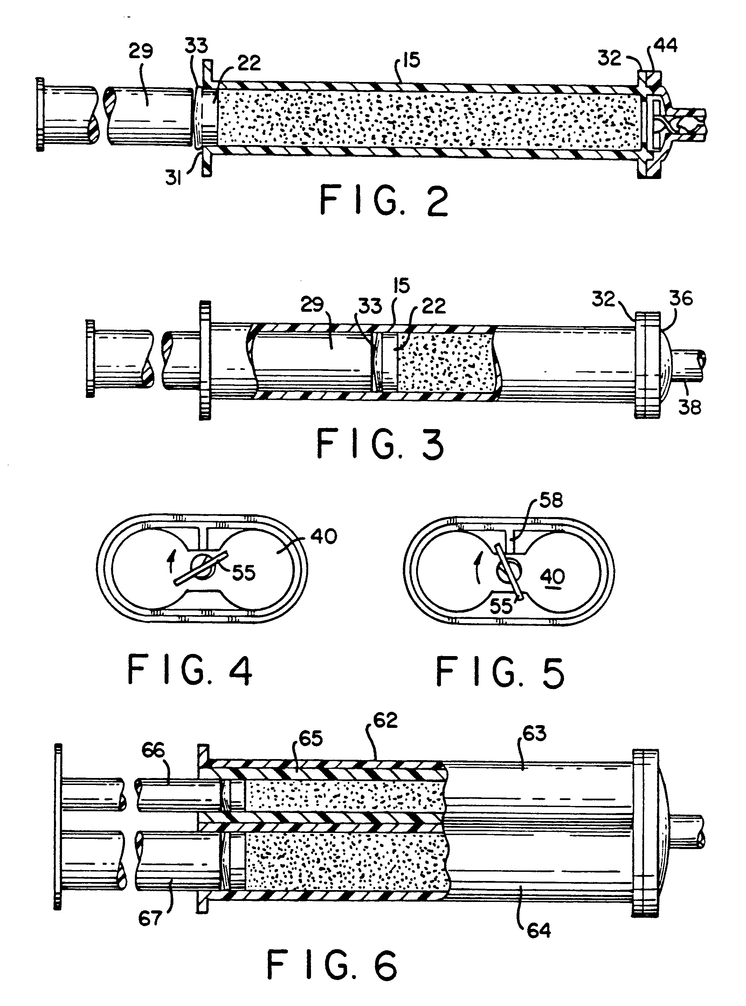

The sealing caps (22) and (24) are removably inserted into the compartments (15) and (16) at the distal end (31) of the syringe (12) after each of the compartments (15) and (16) is filled with a desired material composition. Each sealing cap (22) and (24) has a resilient annular shoulder (33), as best shown in Figures 2 and 3, which is located about its periphery to permit the sealing caps (22) and (24) to seal the compartments and to slidably move along the chamber walls of the compartments (15) and (16) in response to a corresponding movement of the pistons (29) and (30). The annular shoulder (33) prevents backflow of material while the pistons (29) and (30) are advancing.

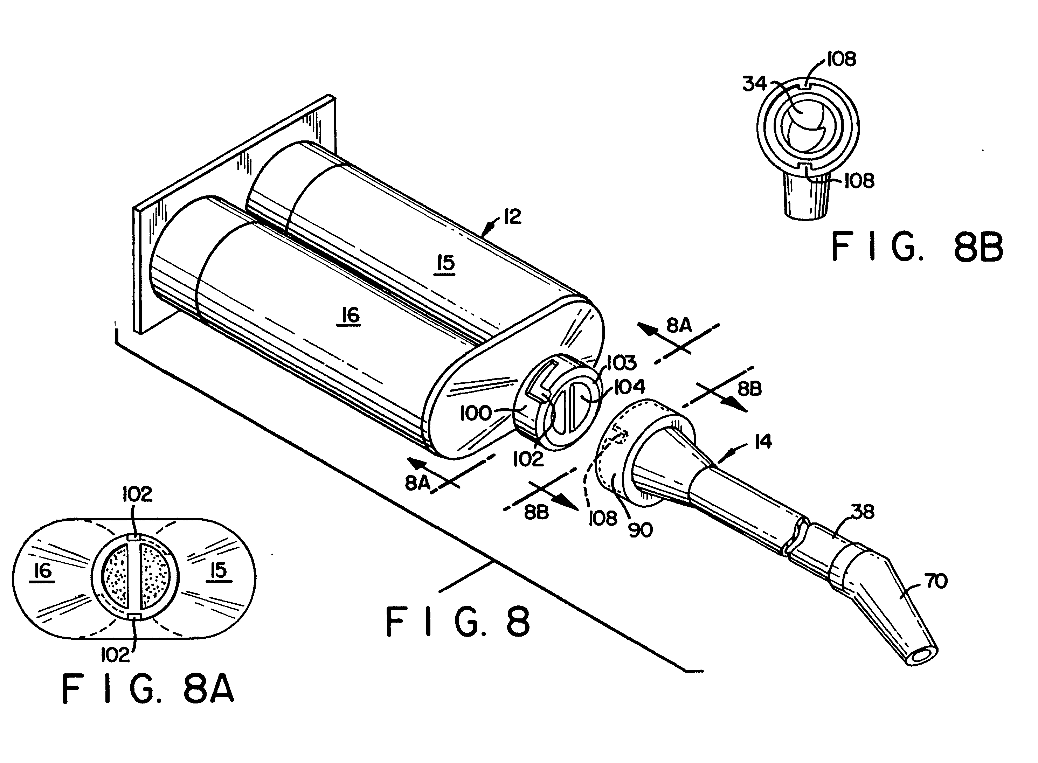

A collar (32) is molded about the body of the syringe (12) for coupling the syringe (12) to the nozzle assembly (14). The collar (32) is recessed from the proximal end (25) of the syringe (12) to form a ledge (35) which is slidably inserted into the head (36) of the nozzle assembly (14) with the collar (32) abutting a rim (44) extending from the head (36). The rim (44) may be either permanently affixed to the collar (32) or mechanically secured thereto. A permanent connection may be made by ultrasonically welding the abutting ends together or by bonding the ends using an adhesive. Alternatively, the syringe (12) may be removably coupled to the nozzle assembly (14) using the arrangement shown in Figure 8.

When the syringe (12) is permanently affixed to the nozzle assembly (14), the dispensing mixer (10) is intended to be disposable and thrown away upon exhausting the supply of stored material in the syringe (12). With the syringe (12) mechanically interlocked to the nozzle assembly (14) as shown in Figure 8) the nozzle assembly (14) may be reused and a different syringe (12) substituted for the spent one. The Figure 8 arrangement permits the nozzle assembly (14) to be readily disconnected from the syringe (12) after use, and reconnected to a new syringe.

The nozzle assembly (14) further includes a static mixer (37) and a nozzle (38) which extends axially outwardly and then tapers down to a discharge opening (39). The nozzle (38) has a central bore (42) which extends axially from the head (36) to the distal end (54) at the nozzle (38). The head (36) of the nozzle assembly (14) has a depressed area (40) which forms a marginal well into which material may flow and merge before entering into the bore (42) of the nozzle (38).

The static mixer (37) of the nozzle assembly (14) consists of a multiple number of serially arranged blades (45) which have a bow tie-like configuration. Each blade (45) is twisted so that its upstream and downstream edges (47) and (48), respectively are at a substantial angle to each other and with each adjacent successive blade (45) twisted in an opposite direction with respect to its preceding blade. It is now well known that if a static mixer (37) is held in a stationary position in a nozzle, it will cause substantial intermixing of any two component fluids when forced through such nozzle.

The static mixer (37) is arbitrarily inserted into the bore (42) of the nozzle (38) so that its forward blade (52) will nestle against the distal end (54) of the nozzle (38). The static mixer (37) has an arm (55) extending from the rearward end thereof which lies substantially transverse to the longitudinal axis of the nozzle (38).

The static mixer (37) is rotated into a fixed position from any initial arbitrary position as is depicted in Figures 4 and 5. The position shown in Figure 4 represents an arbitrary placement of the static mixer (37) in the nozzle (38). The discharge of material from compartments (15) and (16) of the syringe (12) causes the static mixer to turn until the arm (55) engages the end stop (58) as shown in Figure 5. This occurs almost immediately following the ingress of the storage materials into the nozzle (38). The end stop (58) prevents further rotation of the arm (55) as material is fed into the nozzle (38), thereby allowing the mixer to intermix the two materials from the separate storage compartments (15) and (16).

A turning force is initiated to rotate the static mixer (37) due to the geometrical configuration of the serially arranged blades (45) extending from the static mixer (37). However, once the static mixer (37) is rendered immobile, the materials fed through the mixer (37) intermix to from a relatively homogeneously mixed product.

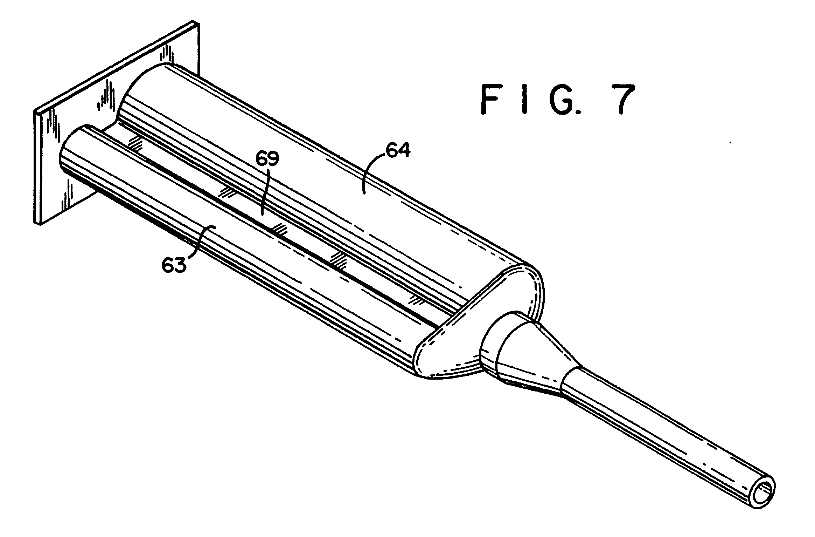

Since the compartments (15) and (16) in the syringe (12) of Figure 1 are of equal size and the pistons (29) and (30) move in unison, the volume of material displaced in each compartment (15) and (16) will be equally independent of the material viscosity and independent of any difference in viscosity. In the embodiment of Figure 6, a syringe (62) is shown corresponding to the syringe (12) of Figure 1, except that the compartments (63) and (64) are of unequal diameter. This can be accomplished by changing the diameter of the compartment (63) relative to the compartment (64) using, for example, a removable liner (65). This changes the mixing ratio of the materials discharged form the compartments (63) and (64), respectively. This pistons (66) and (67) must, however, also be sized corresponding to the selected diameters for the compartments (63) and (64). The use of different volumes for the compartments provides control over the volumetric ratio of discharged material from the syringe (62). The preferred syringe design for use with different diameter compartments is shown in Figure 7. The compartments (63) and (64) are separated by a web of material (69). This provides a greater degree of separation between the compartments (63) and (64) and avoids the need for close manufacturing tolerance.

The discharge opening (39) of the nozzle (38) may be varied using a closed taper which the operator can snip off to control the size of the discharge opening (39). Alternatively, a nozzle tip (70) as shown in Figure 8 may be used.

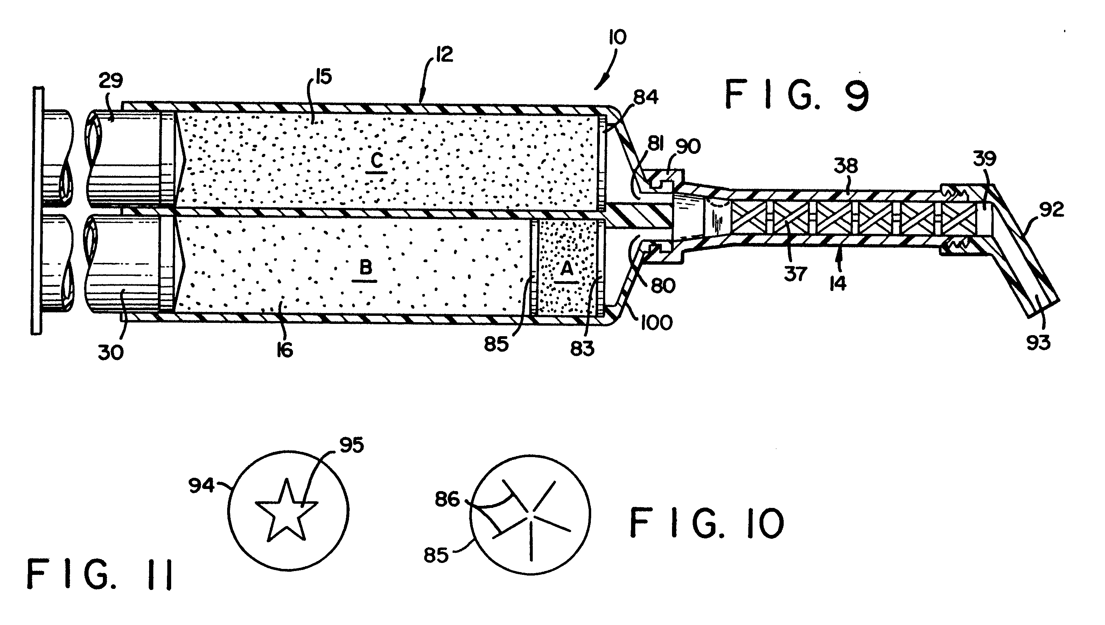

The multi-barrel dispenser (10) may also be used to store and dispense more than one viscosity impression material. For example, two elastomeric, self-curing, impression materials of different viscosities each having a base or a catalyst constituent (A) and (B), or conversely, (B) and (A), and a corresponding catalyst or base (C) may be incorporated into the syringe (12) as shown in Figure 9. The components from each compartment are fed in common into the common nozzle assembly (14) where they intermix before discharge in the same manner as discussed heretofore in connection with Figures 1 and 8. Each impression material is discharged in common with a catalyst and in sequence with each other.

Each of the storage compartments (15) and (16) has a discharge opening (80) and (81) for expelling impression material through the head (82) of the syringe (12). A pair of thin diaphragm membranes (83) and (84) may be used to initially close off each of the discharge openings (81) and (82). The membranes (83) and (84) are normally closed members which readily open in response to a predetermined minimum driving force applied to each compartment from a pair of plungers such as (29) and (30). A diaphragm member (85) as shown in Figure 10 having closed score lines (86) may be used for the membranes (83) and (84), respectively.

The nozzle assembly (14) includes a removable head (90), a common nozzle (38), a removable spout (92) and a static mixing element (37). The nozzle (38) extends from the head (90) to the removable spout (92). The spout (92) is threadably coupled to the nozzle (38) for easy replacement. Although the spout (92) is shown in Figure 9 with internal threads, it may be externally threaded and the nozzle (38) internally threaded. The spout (92) is preferably tapered to provide a predetermined size discharge opening (93), which may have any desired cross-sectional shape and may also be angled.

The storage compartments of the dispenser (10) are preloaded with elastomeric impression materials in a predetermined manner to form predetermined unit dosages, as hereafter explained. The compartment (16) is arbitrarily loaded with the base impression materials (A) and (B), or (B) and (A), respectively, with the base materials (A) and (B) separated by a diaphragm such as 85. Compartment (15) is loaded with a catalyst (C) for use in common with each of the base materials (A) and (B) or alternatively with a separate catalyst for each base impression material. The diaphragm (85) opens in response to a predetermined driving pressure for discharging material from each compartment in common.

The elastomeric impression materials are selected from any known self-curing materials, preferably a silicone elastomeric impression material consisting basically of a diorganopolysiloxane such as divinylpolysiloxane and an organosilicon crosslinker, preferably containing silicone bound hydroxyl groups. The catalyst is preferably platinum siloxane complexes.

The base impression material (A) is a low viscosity material for directly coating the intraoral site whereas impression material (B) is of a substantially higher viscosity material. The base impression material (B) is loaded into compartment (16) containing impression material (A) in a tandem arrangement separated by the diaphragm (85), having the configuration as shown in Figure 10 or by a thin wafer-like diaphragm (94) as shown in Figure 11. The diaphragm (94) has an opening (95), preferably centrally located, to allow the more viscous impression material (B) to be discharged following the discharge of impression material (A). The diaphragm (85) separating the base impression materials (A) and (B) is primarily intended to prevent splashback of the low viscosity material (A) when the high viscosity material (B) is loaded into compartment (16). It also prevents any significant intermixing of the high and low viscosity materials when pressure is applied to drive the impression materials through the discharge openings (80) and (81).

The quantity of the base impression materials (A) and (B) are premeasured to provide predetermined unit dosages when mixed with catalyst (C).

The base impression materials and catalyst are driven from the compartments (15) and (16) by plungers (29) and (30) or by any other conventional drive mechanism. The plungers (29) and (30) are preferably coupled together to be driven in unison by, e.g., a conventional double-barreled, ratchet-type gun (not shown), which may be mechanically or automatically activated.

The dispenser (10) is operated to discharge the mixed impression materials (A) and (B) in sequence with the low viscosity material extruded intraorally directly upon the patient's teeth through the spout (92), followed by extrusion of the higher viscosity material directly in the dental tray. It is preferable to remove the spout (92) after the low viscosity material is fully extruded. Upon removal of the spout (92), the discharge opening (39) of the nozzle (14) provides an opening which is larger than the orifice (93) of the spout (92) permitting the more viscous impression material to be readily extruded into the dental tray. The impression materials may also be of different colors to simplify the process for the dentist. In this fashion, the dentist can complete an impression under controlled aseptic conditions in an efficient manner.

The spout (92) may be replaced with a spout having a rectangular cross section to provide a flat ribbon of impression material for use intraorally. By placing impression material on the biting surface of the teeth, the patient, upon closing the upper and lower teeth, forms an intraocclusal record. This may be used by the dental laboratory to relate the opposing case models during fabrication of the prosthesis, so that proper contour and interdigitation can be accomplished.

In the dispensing apparatus (10) of Figure 9, the syringe (12) is removably coupled to the nozzle assembly (14) using the closure arrangement of Figure 8. The syringe (12) has a cylindrical head (100) with a recessed groove (102) located symmetrically on opposite sides of the head (100). Each groove (102) is aligned parallel to the longitudinal axis of the syringe and extends from a chamfer (103) at the mouth (104) of the syringe (12) to a connecting recess (105) which circumscribes an arc lying in a plane, substantially transverse to the groove (102) or at a small acute angle relative thereto. The nozzle assembly (14) has a complementary cylindrical head (90) extending from the nozzle (38). The diameter of the head (90) is slightly larger than the diameter of the corresponding head (100). The head (90) has two tong-like projections (108) on opposite sides which are adapted to fit into the recessed grooves (102). To assemble the head of the nozzle into the syringe, the head (90) is positioned over the head (100) with the tongs (108) aligned with the recessed grooves (102). The tong-like projections are slid down into the grooves (102) until the tong-like projections (108) reach the curved recess (105), at which time the head (100) is rotationally twisted to lock the projections (108) into the curved recess (105). This provides a pressure lock which can withstand the forces generated when dispensing impression material.

## Claims

1. A dispensing mixer for storing, intermixing and dispensing at least two materials from a common nozzle comprising:
a syringe having at least two compartments for separately storing each of the materials to be intermixed;
discharge means for simultaneously discharging material from each end of said compartments;
a diaphragm member located in each compartment at one end thereof for securing the material stored in each compartment, respectively, with each diaphragm adapted to open in response to a predetermined applied pressure from said discharge means; and
a nozzle assembly comprising said common nozzle with said nozzle having a bore for receiving the materials dispensed from said syringe and a head extending from said nozzle for coupling said nozzle assembly to said syringe, a static mixing element disposed within the bore of said nozzle for intermixing the materials fed to said nozzle, said static mixing element having an arm at one end thereof for engaging an end stop projecting from said head to render said static mixer immobile in response to the ingress of material fed through said nozzle.

2. A dispensing mixer, as defined in claim 1, wherein each diaphragm member has a multiple number of sections which open in response to pressure from said discharge means and close upon the removal of pressure to self-lock each compartment.

3. A dispensing mixer, as defined in claim 2, wherein said syringe has a collar for engaging said head to couple said nozzle assembly to said syringe.

4. A diaphragm mixer, as defined in claim 3, wherein said collar is permanently secured to said head to form a disposable mixer adapted to the thrown away upon discharging the contents from each compartment.

5. A dispensing mixer, as defined in claim 3, wherein said collar is detachably coupled to said head.

6. A dispensing mixer, as defined in claim 5, wherein said head is in the form of a clamp for removably engaging the collar from said syringe.

7. A dispensing mixer, as defined in claim 5, further comprising a sealing cap for each compartment, with each sealing cap having an annular shoulder for slidably moving within each chamber in response to the movement of said discharge means.

8. A dispensing mixer, as defined in claim 7, further comprising means for changing the mixing ratio between the materials from each compartment.

9. Apparatus for dispensing a unit dosage of elastomeric impression material for taking an impression in the oral cavity under aseptic conditions in the preparation of a dental restoration comprising:

a syringe having a common head and two elongated storage compartments laterally spaced apart and extending from said head, with each compartment having a discharge end terminating in said common head;

means for simultaneously applying pressure upon the opposite end of each compartment for discharging material from the discharge end of each compartment in common; and

a nozzle assembly having a single nozzle for dispensing the materials discharged from said syringe, a static mixing element in said nozzle for intermixing the materials fed to said nozzle, and means for removably coupling said nozzle assembly to said syringe with said means comprising a cylindrical member having tong-like projections for engaging the head of said syringe, and wherein the head of said syringe includes symmetrically disposed recessed grooves in alignment with the longitudinal axis of said syringe, and a curved recess in a plane substantially transverse thereto into which said tong-like projections are removably placed.

FIG. I

EP 0 319 639 A1

FIG. 2

FIG. 3

FIG. 4

FIG. 5

FIG. 6

F I G. 7

63

69

64

EP 0 319 639 A1

FIG. 8B

FIG. 8

FIG. 8A

FIG. 9

C

B

A

29  15  12  10  84  81  90  38  39  92

30  16  85  83  100  80  37  14  93

FIG. 11

94  95

86  85

FIG. 10

EP 0 319 639 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 767 085  (CANNON)<br>* Description; figures * | 1-7,9 | A 61 C   9/00 |
| X | EP-A-0 121 342  (MINNESOTA MINING AND MANUFACTURING)<br>* Abstract; figures * | 1,3-7,9 | |
| X | EP-A-0 105 181  (LIQUID CONTROL INTERNATIONAL CO.)<br>* Description; figures * | 1,3-9 | |
| A | US-A-3 330 444  (RAYPHOLTZ)<br>* Figures 1-3 * | 8 | |

TECHNICAL FIELDS
SEARCHED (Int. Cl.4)

A 61 C

The present search report has been drawn up for all claims

| Place of search | Date of completion of the search | Examiner |
|---|---|---|
| THE HAGUE | 08-03-1989 | VANRUNXT J.M.A. |

EPO FORM 1503 03.82 (P0401)